Europäisches Patentamt

European Patent Office

Office européen des brevets

(11) Veröffentlichungsnummer: **0 119 301**
**A2**

(12) # EUROPÄISCHE PATENTANMELDUNG

(21) Anmeldenummer: 83111381.6

(22) Anmeldetag: 14.11.83

(51) Int. Cl.³: **C 07 D 307/60**

(30) Priorität: 17.02.83 IT 1963783

(43) Veröffentlichungstag der Anmeldung: 26.09.84
Patentblatt 84/39

(84) Benannte Vertragsstaaten: **AT BE CH DE FR GB LI LU NL SE**

(71) Anmelder: **ALUSUISSE ITALIA S.p.A., Via Vittor Pisani, 31, I-20124 Milano (IT)**

(72) Erfinder: **Verde, Luigi, Viale Stelvio 96, Busto Arsizio (Prov. Varese) (IT)**
Erfinder: **Neri, Amleto, Via Cifrondi 14, Bergamo (IT)**
Erfinder: **Moreno, Sergio, Via Martin Luther King 10, Biassono (Prov. Milano) (IT)**

(74) Vertreter: **von Füner, Alexander, Dr. et al, Patentanwälte v. Füner, Ebbinghaus, Finck Mariahilfplatz 2 & 3, D-8000 München 90 (DE)**

(54) Verfahren und Vorrichtung zur Maleinsäureanhydridgewinnung aus den Abgasen von Phthalsäureanhydridanlagen.

(57) Es wird ein Verfahren zur Gewinnung von als Nebenprodukt in den Abgasen von Phthalsäureanhydridanlagen enthaltenem Maleinsäureanhydrid beschrieben. Dieses Verfahren besteht aus einer Gaswäsche, um eine wäßrige Lösung der Maleinsäure und der übrigen im Gas enthaltenen organischen Substanzen einschließlich Phthalsäure zu erhalten, einer Anreicherung dieser Lösung als Maleinsäure bis zu einer Konzentration von 25–35 Gew.-% durch Zugabe eines Rückführungsstroms zur Lösung sowie Entfernung der Phthalsäure durch Kristallisation. Die erhaltene, maleinsäurereiche und an Phthalsäure verarmte Lösung wird dann teilweise in die Gaswaschstufe (um die Löslichkeit der Phthalsäure zu fördern und die so erhaltene Lösung an Maleinsäure anzureichern) zurückgeführt und teilweise einer Behandlung zur Gewinnung des Maleinsäureanhydrids durch Dehydratation mittels azeotroper Destillation mit ortho-Xylol unterzogen. Das entstandene rohe Maleinsäureanhydrid wird ann durch Rektifizierung in Bodenkolonnen gereinigt.

Ferner wird eine Vorrichtung zur Gewinnung von Maleinsäureanhydrid beschrieben, welche einen Gaswaschteil, einen Maleinsäuredehydratationsteil und einen Maleinsäureanhydridreinigungsteil umfaßt.

# Verfahren und Vorrichtung zur Maleinsäureanhydridgewinnung aus den Abgasen von Phthalsäureanhydridanlagen

Vorliegende Erfindung betrifft ein Verfahren und eine entsprechende Vorrichtung zur Gewinnung des als Nebenprodukt in Phthalsäureanhydridanlagen erhaltenen Maleinsäureanhydrids.

In Phthalsäureanhydridherstellungsverfahren durch katalytische Dampfphasenoxydation von ortho-Xylol und/ oder Naphthalin mit Luft enthalten die Reaktorabgase nach Kühlung und Abtrennung des Phthalsäureanhydrids durch Desublimation einerseits Stickstoff, Sauerstoff, Wasser und Verbrennungsprodukte ($CO$ und $CO_2$) sowie andererseits verschiedene organische Substanzen, bei denen es sich hauptsächlich um in den Desublimatoren nicht isoliertes Phthalsäureanhydrid und einige Nebenprodukte der Reaktion, insbesondere Maleinsäureanhydrid und geringere Mengen Citraconsäureanhydrid, ortho-Toluylaldehyd, Benzoesäure, Chinone und geringfügigere Verunreinigungen handelt.

Maleinsäureanhydrid liegt in solchen Abgasen üblicherweise in einer Konzentration von 0,2 - 0,3 Gew.-% bezogen auf das Gesamtgewicht der Gase vor. Im Hinblick auf den nicht zu vernachlässigenden Prozentsatz, zu dem diese Verbindung vorliegt, und auf die Tatsache, dass diese Verbindung einen wertvollen Rohstoff zur Herstellung von Harzen und höher entwickelten Produkten darstellt, ergibt sich einerseits ein wirtschaftliches Interesse daran, das Maleinsäureanhydrid als solchen wertvollen Rohstoff zu gewinnen, und andererseits die Notwendigkeit dieser Gewinnung, um die Umweltbelastung durch die aus der Phthalsäureanhydridherstellungsanlage abgelassenen Gase zu verringern.

Es wurden schon verschiedene Maleinsäureanhydridgewinnungsverfahren vorgeschlagen, worunter das in der U.S. Patentschrift 3 642 892 angegebene die grösste Bedeutung hat. Bei diesem Verfahren handelt es sich um eine Wäsche der Abgase, um die darin enthaltenen organischen Substanzen in Form einer wässrigen Lösung zu erhalten, die nachfolgende Entfernung des Wassers in einer Eindampfungsstufe unter kontrollierten Bedingungen, um jegliche Kristallisation der vorhandenen organischen Verbindungen zu verhindern, danach deren Dehydratation und die Gewinnung des Maleinsäureanhydrids durch Destillation und selektive Kondensation. Die bisher vorgeschlagenen Verfahren, insbesondere das von der Firma U.C.B. angemeldete, arbeiten mit Maleinsäure/Phthalsäureverhältnissen in den aus der Gaswäsche austretenden Lösungen, welche im wesentlichen den in den zu waschenden Gasen bestehenden und somit verhältnismässig niedrigen Verhältnissen der Grössenordnung von 12 - 15 bis 2 (Gew./Gew.) entsprechen.

Solche Verhältnisse erfordern einen Betrieb bei verhältnismässig hohen Verdünnungen der organischen Substanzen in den verschiedenen Lösungen, um eine Ausfällung durch Unlöslichkeit der Phthalsäure selbst zu verhindern. Dies bedeutet, dass man bei den bekannten Verfahren beträchtliche Mengen Verdünnungswasser im Kreislauf führen muss, welche erheblichen Energieverbrauch in den nachfolgenden Stufen der Entfernung des Wassers zur Dehydratation des Produkts und der Gewinnung des Maleinsäureanhydrids erfordern.

Es ist die Hauptaufgabe dieser Erfindung, ein Verfahren zur Maleinsäureanhydridgewinnung aus den Abgasen von Phthalsäureanhydridanlagen bereitzustellen, bei dem Probleme mit Verstopfungen im Innern der Produktionsstrasse verhindert und die Gefahr der Ausfällung im Verfahren umlaufender organischer Verbindungen beseitigt wird.

Eine weitere Aufgabe der vorliegenden Erfindung besteht darin, die Verwendung höher konzentrierter Maleinsäurelösungen zu ermöglichen und damit die Notwendigkeit einer späteren Entfernung von beträchtlichen Mengen Verdünnungswasser und somit erhöhten Energieverbrauchs zu vermeiden.

Nicht zuletzt ist es eine Aufgabe dieser Erfindung, ein Verfahren bereitzustellen, welches die Gewinnung von Maleinsäureanhydrid in hoher Ausbeute und Reinheit ermöglicht.

Schliesslich ist es Aufgabe der vorliegenden Erfindung, eine zur praktischen Durchführung des besagten Verfahrens zur Gewinnung von Maleinsäureanhydrid geeignete Vorrichtung anzugeben.

Diese sowie weitere, weiter unten ersichtliche Aufgaben werden durch das erfindungsgemässe Verfahren zur Maleinsäureanhydridgewinnung aus den Abgasen von Phthalsäureanhydridanlagen, bei dem diese Gase hauptsächlich aus Maleinsäureanhydrid, Phthalsäureanhydrid, Citraconsäureanhydrid und Chinonen bestehende organische Substanzen enthalten und welches eine Wäsche dieser Gase zur Abscheidung jener organischen Substanzen in Form einer wässrigen Lösung der entsprechenden Säuren und die Gewinnung von Maleinsäureanhydrid aus dieser sauren Lösung umfasst, gelöst, welches dadurch gekennzeichnet ist, dass man stufenweise:

a) diese saure Lösung durch Zusatz eines an Maleinsäure angereicherten und an Phthalsäure verarmten Rückführungsstroms in jene Waschstufe an Maleinsäure bis zu einem Maleinsäure/Phthalsäuregewichtsverhältnis von 17:1 bis 20:1 und zu einer Maleinsäurekonzentration von 25 – 35 Gew.-% bezogen auf diese saure Lösung anreichert,

b) die Phthalsäure durch Vakuumkristallisation und Filtrieren aus dieser angereicherten Lösung entfernt,

c) einen Teil dieser aus Schritt (b) kommenden angereicherten Lösung als solchen Rückführungsstrom in den Schritt (a) zurückleitet,

d) den verbleibenden Teil dieser angereicherten Lösung aus Schritt (b) einer Behandlung zur Gewinnung von Maleinsäureanhydrid unterzieht und

e) das in Schritt (d) erhaltene Maleinsäureanhydrid reinigt.

Gemäss einem weiteren Gegenstand vorliegender Erfindung werden die oben erwähnten Aufgaben durch eine erfindungsgemässe Vorrichtung zur Maleinsäureanhydridgewinnung aus den Abgasen von Phthalsäureanhydridanlagen gelöst, welche aus einem Gaswaschteil, einem Maleinsäuredehydratationsteil und einem Maleinsäureanhydridreinigungsteil besteht und dadurch gekennzeichnet ist, dass

A) dieser Waschteil mindestens eine Waschkolonne zur Gegenstromwäsche dieser Gase mit wässriger Lösung und rückgeführter, an Maleinsäure angereicherter Lösung, mindestens einen mit Evakuierungsmitteln und Rührwerk versehenen Kristallisator zur Kristallisation der Phthalsäure und mindestens ein Filter zur Entfernung der kristallisierten Phthalsäure sowie Einrichtungen zur teilweisen Rückführung der aus diesem Filter austretenden, angereicherten Lösung in jene Waschkolonne umfasst,

B) dieser Maleinsäuredehydratationsteil mindestens eine Destillationskolonne mit Böden, einen Kessel vor dieser Kolonne und einen Kondensator sowie eine Vorlage nach dieser Kolonne und Mittel zum Evakuieren dieser Kolonne umfasst und .

C) dieser Maleinsäureanhydridreinigungsteil mindestens zwei hintereinandergeschaltete Rektifizierkolonnen mit Böden, mindestens eine Pumpe zur Förderung von Sumpfprodukt aus dieser zweiten Rektifizierkolonne als Rückfluss auf den Kopf dieser ersten Rektifizierkolonne, Mittel zum Evakuieren dieser Rektifizierkolonnen und einen Kessel vor sowie einen Kondensator und eine Vorlage nach diesen beiden Rektifizierkolonnen umfasst.

Weitere Merkmale und Vorzüge dieser Erfindung sind beispielhaft aus der detaillierten Beschreibung bevorzugter Ausführungsformen des erfindungsgemässen Verfahrens bzw. der Vorrichtung anhand der beigefügten Zeichnungen näher ersichtlich. Es zeigen:

Fig. 1 ein Fliessschema des erfindungsgemässen Waschteils und

Fig. 2 ein Fliessschema des erfindungsgemässen Maleinsäuredehydratationsteils und Maleinsäureanhydridreinigungsteils.

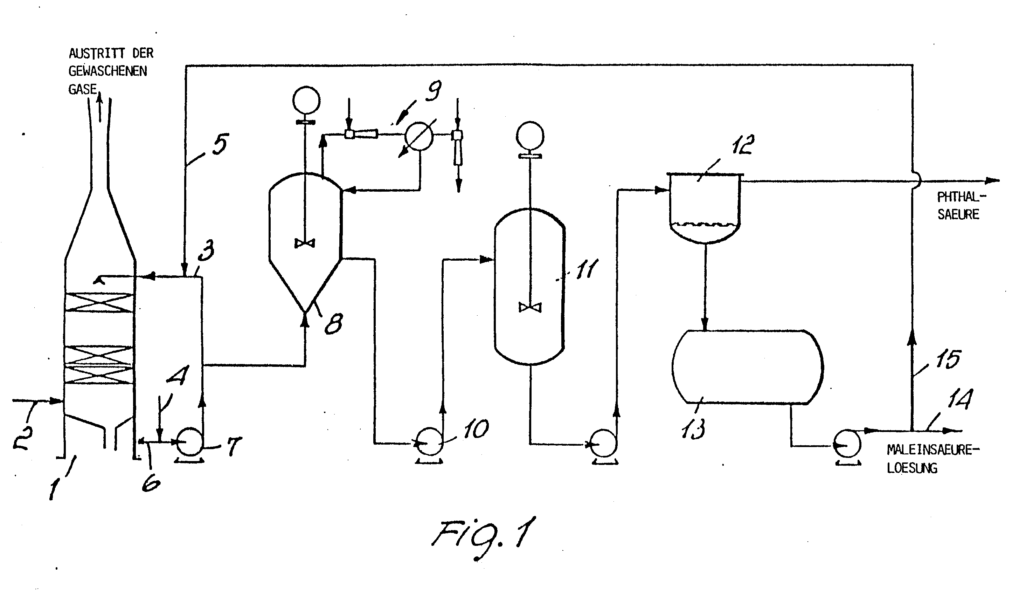

Mit Bezug auf die oben erwähnten Zeichnungen umfasst das erfindungsgemässe Verfahren eine erste Stufe, bei der die aus einer Phthalsäureanhydridanlage kommenden Abgase gewaschen werden und die gemäss einer bevorzugten Ausführungsform in einem Waschteil wie in Figur 1 abgebildet durchgeführt wird.

Die besagten Gase enthalten die oben erwähnten organischen Substanzen im allgemeinen in folgenden Prozentsätzen: Maleinsäureanhydrid 0,2 - 0,3%, Phthalsäureanhydrid 0,02 - 0,03%, Citraconsäureanhydrid 0,01 - 0,03% sowie Benzoesäure, Chinone und weitere organische Begleitsubstanzen 0,02 - 0,03%, wobei diese Prozentangaben als Gewicht/Gasgesamtgewicht ausgedrückt sind. Im allgemeinen erhält man nach dem Waschen dieser Gase Lösungen der organischen Substanzen, worin die relativen Anteile der vorhandenen Verbindungen im wesentlichen diejenigen im Gas vor der Wäsche widerspiegeln.

Zweckmässig erfolgt nun erfindungsgemäss in der Waschstufe eine Anreicherung an Maleinsäure in der wässrigen Lösung der aus den Gasen abgeschiedenen organischen Substanzen. Dafür werden die aus der Phthalsäureanhydridanlage kommenden Gase über die Leitung 2 in eine Waschkolonne (Skrubber) 1 eingeleitet, die von oben zur Gegenstromwäsche der Gase mit einer wässrigen Waschlösung beschickt wird. Die Waschlösung in den als 3 bezeichneten Leitungen wird ausser mit dem Frischwasser über Leitung 4, das den Verdunstungsverlusten sowie dem Hydratationswasser und dem Verdünnungswasser für die in den in die Atmosphäre abgelassenen gewaschenen Gasen enthaltenen organischen Substanzen entspricht, mit einem wässrigen maleinsäurereichen und an Phthalsäure verarmten, aus der Leitung 5 kommenden Rückführungsstrom vereinigt. Die Waschkolonne 1 ist im Innern mit solchen Böden und Füllkörpern ausgerüstet, dass intimer Kontakt zwischen den Gasen und der wässrigen Waschlösung bei geringster Verschmutzung mit ausgefällten Substanzen und bei kleinstem Druckverlust ermöglicht wird.

Die Lösung, welche die aus dem Gas in Form der entsprechenden Säuren abgeschiedenen organischen Substanzen enthält, verlässt den Sumpf der Kolonne 1 über die Leitung 6. Ein Teil dieser Lösung wird über die Pumpe 7 und die Leitung 3 als Rückfluss auf die Kolonne 1 zurückgeführt, während der restliche Teil in einen Kristallisator 8 gelangt. Dieser ist mit einem Rührwerk und einem Evakuierungssystem versehen, welches von Dampfstrahlsaugern bekannter Art oder anderen gleichwertigen, allgemein als 9 bezeichneten Systemen gebildet wird. Im Kristallisator 8 erfolgt ein rascher Temperaturabfall durch Entspannung, und wenn die Kristallisationstemperatur etwa
10°C bei einem Druck von etwa 10 - 15 Torr erreicht,
scheidet sich ein Grossteil der Phthalsäure aus, wobei
nur der bei dieser Temperatur lösliche Anteil (etwa 0,3
Gew.-% bezogen auf die Lösung der Säuren) in Lösung bleibt.

Vom Kristallisator gelangt die saure Lösung mittels einer Pumpe 10 zu einer Filtereinrichtung zur Entfernung der ausgefällten Phthalsäure. Vorzugsweise ist
den Filtern ein Homogenisierungsgefäss 11 vorgeschaltet,
das es ermöglicht, saure Lösung für einen gegebenenfalls
diskontinuierlichen Filtrierbetrieb zu speichern. Aus
dem Homogenisator wird die Lösung einem Filter 12 zugeführt, wo die suspendierte Phthalsäure zurückgewonnen und
zur nachfolgenden Dehydratation und Gewinnung als Anhydrid
in die Phthalsäureanhydridanlage zurückgeleitet wird.

Die so erhaltene filtrierte Lösung ist nun maleinsäurereich und an Phthalsäure verarmt. Diese Lösung wird
in einem Behälter 13 gespeichert, von wo ein Teil davon
als besagter Rückführungsstrom über die Leitung 5 zur
Waschkolonne 1 zurückgeleitet wird, um die Phthalsäure in
der durch den Waschvorgang gebildeten sauren Lösung löslich
zu machen.

Die Menge der durch die Leitung 5 laufenden Rückführungslösung wird so gesteuert, dass man in der aus der
Waschkolonne 1 austretenden sauren Lösung eine
Maleinsäurekonzentration von 25 - 35 Gew.-% und ein Male-
insäure/Phthalsäureverhältnis von 17 - 20/1 Gew./Gew. in
dieser Lösung erhält.

Der verbleibende, eine Maleinsäurekonzentration
von 25 - 35 Gew.-% aufweisende Teil der sauren Lösung aus
dem Behälter 13 wird entweder direkt in die nachfolgende
Maleinsäureanhydridgewinnungsstufe oder über die Leitung
14 zum Lager gefördert.

Wie sich aus der obigen Beschreibung der ersten
Stufe des erfindungsgemässen Verfahrens ergibt, arbeitet
man mit erhöhten Maleinsäureanhydrid/Phthalsäureanhydridverhältnissen, wodurch der Skrubber 1 bei höheren Malein-

säurekonzentrationen betrieben werden kann, ohne dass Fällungsprobleme wegen der Löslichkeit der Phthalsäure selbst auftreten. Mit dieser Massnahme erzielt man mehrere erhebliche Vorteile:

a) man vermeidet Probleme mit der Kristallisation der Phthalsäure im Innern der Anlage im Waschteil, was Verstopfungsprobleme an den Einbauten der Waschkolonne (Böden und Füllkörper) beseitigt;

b) ein Nebenprodukt (Phthalsäure) wird aus der Lösung entfernt und zurückgewonnen, wodurch die Phthalsäureanhydridausbeute erheblich erhöht wird; und

c) man kann bei höheren Maleinsäurekonzentrationen arbeiten, was die Zufuhr von Verdünnungswasser und somit die Notwendigkeit späterer Entfernung erheblicher Mengen an Verdünnungswasser, die grösseren Energieverbrauch zur Folge hätten, in der Maleinsäureanhydriddehydratations- und Gewinnungsstufe begrenzt.

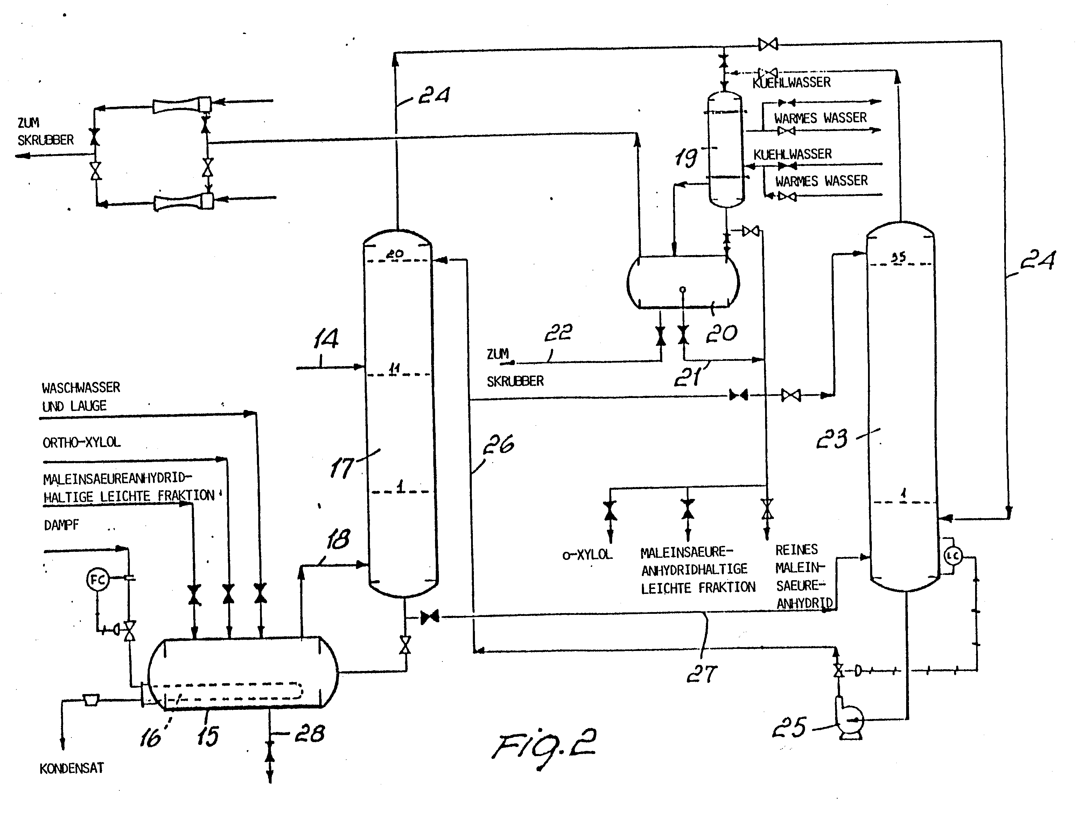

Die entweder direkt von der Filtration über die Leitung 14 oder gegebenenfalls aus der Zwischenlagerung kommende Maleinsäurelösung wird nun einer Behandlung zur Gewinnung von Maleinsäureanhydrid unterzogen, die mit Bezug auf Figur 2 erläutert sei.

Zur Gewinnung von Maleinsäureanhydrid ist im wesentlichen eine Dehydratations- und Wasserentfernungsstufe vorgesehen, die nach einem an sich bekannten azeotropen Destillationssystem mit ortho-Xylol durchgeführt wird.

Das ortho-Xylol wird in einen durch einen beispielsweise dampfbetriebenen Heizer 16 erhitzten Kessel 15 eingeführt. Im Kessel wird das ortho-Xylol bei Atmosphärendruck bis auf eine Temperatur von etwa 140°C. erhitzt. Danach beginnt man, die Maleinsäurelösung in eine Destillationskolonne 17 mit Böden, im allgemeinen einer Bodenzahl von 15 bis 25, vorzugsweise 20 Böden, einzuleiten. In diesem letzten Fall wird die Maleinsäurelösung in Höhe des elften Bodens der Kolonne 17 eingeführt. Die aus dem Kessel 15 über die Leitung 18 kommenden ortho-Xyloldämpfe bilden mit dem in der Maleinsäurelösung eintretenden Wasser sowie dem Dehydratationswasser aus der Maleinsäure selbst ein Wasser/ortho-Xylolgemisch, das

gegenüber dem entsprechenden Azeotrop an ortho-Xylol angereichert ist. Das bei einer Temperatur von etwa 110°C abdestillierende Wasser/ortho-Xylolgemisch tritt am Kopf der Kolonne 17 aus und wird im Kondensator 19 gegen einen Kühlwasserkreislauf kondensiert. Die Flüssigkeit aus dem Kondensator 19 sammelt sich in einer Vorlage 20, wo sich das Wasser vom ortho-Xylol durch Dekantieren abtrennen lässt. Das ortho-Xylol wird über die Leitung 21 entnommen und als Rückfluss auf die Kolonne 17 gegeben, während die abgetrennte, Spuren Maleinsäure enthaltende wässrige Phase über die Leitung 22 in die Waschstufe zum Skrubber 1 zurückgeführt wird. Am Ende der azeotropen Destillation verbleiben ortho-Xylol, Maleinsäureanhydrid und weitere aus den behandelten Gasen entfernte organische Substanzen, unter anderem Citraconsäureanhydrid, im Kessel 15. Die Verluste an Maleinsäureanhydrid durch Umlagerung zu Fumarsäure belaufen sich auf etwa 3% des eingesetzten Maleinsäureanhydrids.

Nachdem auch die aus einem vorhergehenden Reinigungszyklus kommenden leichten Fraktionen in den Kessel gegeben wurden, beginnt man mit der Destillation des ortho-Xylols in der Kolonne 17 unter Vakuum bei einem Druck von 250 - 300 Torr und einer Kopftemperatur von etwa 110°C. Das am Kopf abgetrennte ortho-Xylol wird im Kondensator 19 kondensiert und teilweise als Rückfluss auf die Kolonne zurückgegeben und teilweise ins Lager abgeführt.

Der im Kessel 15 verbleibende Rückstand von der ortho-Xyloldestillation besteht aus rohem Maleinsäureanhydrid, das mit Citraconsäureanhydrid, Benzoesäure, Phthalsäureanhydrid und weiteren organischen Verunreinigungen vermischt ist. Darauf schreitet man zur Maleinsäureanhydridreinigungsstufe. Dabei erfolgt eine Rektifizierung in zwei hintereinandergeschalteten Stufen, von denen die erste in derselben, zuvor zur Dehydratations- und Maleinsäureanhydridgewinnungsbehandlung eingesetzten Kolonne 17 und die zweite in einer zweiten Kolonne 23 ebenfalls mit Böden, mit einer Bodenzahl von 30 bis 40, vorzugsweise 35, durchgeführt wird. Beim Betrieb mit einer solchen hohen, über die beiden Kolonnen 17 und 23 verteilten Bodenzahl gelingt die Abtrennung des Maleinsäureanhydrids von den organischen Verunreinigungen, insbesondere den Chinonen und dem Citraconsäureanhydrid mit einem Maleinsäureanhydrid sehr nahe liegenden Siedepunkt, wobei man Maleinsäureanhydrid hoher Reinheit der Grössenordnung von 99,8 Gew.-% erhält, das auch colorimetrische Kenndaten aufweist, die denen von durch Kohlenwasserstoffoxydation erhältlichem Maleinsäureanhydrid äusserst ähnlich sind.

Beim Betrieb mit einer grossen, über zwei Kolonnen verteilten Destillationsbödenzahl erzielt man ferner einen hohen Reinheitsgrad und erhöhte Ausbeuten bei der Wiedergewinnung und erreicht, dass die bereits erwähnte Verwendung titanhaltiger Werkstoffe, die der Korrosion durch organische Verunreinigungen in feuchter Umgebung widerstehen, auf allein die Kolonne 17 beschränkt ist.

In dieser Reinigungsstufe werden die beiden Kolonnen 17 und 23 in Reihe verbunden, wobei die Dämpfe vom Kopf der Kolonne 17 über die Leitung 24 in den Sumpf der Kolonne 23 geführt und das bei 19 kondensierte und in 20 aufgefangene Kopfprodukt der Kolonne 23 teilweise als Rückfluss unter Schwerkraft auf die Kolonne 23 gegeben wird. Die Flüssigkeit aus dem Sumpf der Kolonne 23 wird mittels der Pumpe 25 und der Leitung 26 als Rückfluss auf den Kopf der Kolonne 17 geführt.

In der Anfangsphase der Reinigung wird durch Vakuumdestillation bei etwa 100 Torr und einer Temperatur von 135°C eine restliches ortho-Xylol und Maleinsäureanhydrid enthaltende leichte Fraktion abgenommen; diese Fraktion wird gelagert und bei einem späteren Aufarbeitungszyklus vor der ortho-Xyloldestillationsphase in den Kessel 15 gegeben.

Danach erfolgt die Destillations des Maleinsäureanhydrids bei einem Druck von 100 Torr und einer Temperatur von 135°C; um ein Produkt mit konstanten Kenndaten zu erhalten, muss man das äussere Rücklaufverhältnis ständig erhöhen, das sich von 3,0 bis 3,5 bei Beginn des Destillationsbetriebs auf 9 - 11 bei dessen Ende verändert.

Das am Kopf der Kolonne 23 erhaltene Maleinsäure-

anhydrid wird in 19 mit Wasser kondensiert, das zur Vermeidung des Erstarrungsrisikos auf eine Temperatur oberhalb des Schmelzpunkts des Maleinsäureanhydrids erwärmt ist, und zum Lager geleitet.

Die Verunreinigungen verbleiben im Kessel 15, nämlich Phthalsäureanhydrid, Citraconsäureanhydrid, weitere schwere Produkte und geringere Mengen Maleinsäureanhydrid, dessen weitergehende Rückgewinnung übermässige Rücklaufverhältnisse (und damit Betriebskosten) erfordern würde. Man gewinnt das Maleinsäureanhydrid in einer Ausbeute von etwa 94%, bezogen auf die in den dem vorliegenden Verfahren unterzogenen Gasen vorhandene Menge.

Am Ende des Reinigungsvorgangs wird die Kolonne 17 über die Leitung 27 in den Sumpf der Kolonne 23 entleert, damit das auf den Böden der Kolonne 17 vorliegende Maleinsäureanhydrid im nächsten Reinigungszyklus zurückgewonnen werden kann.

Die abgelassene Flüssigkeit wird dann im Sumpf der Kolonne 23 gelagert, wo sie gekühlt wird, damit das Maleinsäureanhydrid nicht längere Zeit erhöhten Temperaturen ausgesetzt ist.

Die Kolonne 17, der Kessel 15 und der Verdampfer 16 werden mit Wasser und Lauge gewaschen um vor dem nächsten Zyklus sämtliche schweren organischen Verbindungen (insbesondere Fumarsäure und Teere) zu beseitigen. Während dieser Wäsche erwärmt man das System, um die Auflösung der organischen Verbindungen zu fördern. Die dabei erhaltene organische Lösung wird über die Leitung 28 abgezogen und beispielsweise zu einer Verbrennungsanlage geführt.

Aus der obigen Beschreibung sind sämtliche mit dem erfindungsgemässen Verfahren bzw. der Vorrichtung erzielbaren Vorteile ersichtlich. Vor allem gestattet das vorliegende Verfahren die Verwendung höherer Maleinsäurekonzentrationen als 25 Gew.-% und bringt damit eine Energieeinsparung in der nachfolgenden Dehydratationsstufe. Durch die Beseitigung und Rückgewinnung der Phthalsäure vermeidet man die mit der Möglichkeit einer Kristallisation derselben im Inneren der Apparate im Waschteil verbundenen Gefahren.

Die Verwendung von zwei Rektifizierkolonnen mit genügender Bödenzahl erlaubt es, Maleinsäureanhydrid in hohem Reinheitsgrad zu erhalten und die Verwendung von Titan auf nur die erste Rektifizierkolonne 17 zu beschränken. Der mit dem erfindungsgemässen Verfahren erzielbare hohe Reinheitsgrad ermöglicht es, das erhaltene Maleinsäureanhydrid zu tablettieren und damit dessen Lagerung und Transport zu erleichtern: als Feststoff erfordert es keine besonderen Infrastrukturvorkehrungen für den Vertrieb, im Gegensatz zu weniger reinem Maleinsäureanhydrid, das nicht tablettierbar ist und in flüssiger Form gehandhabt werden muss.

Schliesslich erlaubt die Verfügbarkeit von zwei Kolonnen auch die Abtrennung und spätere Wiedergewinnung des auf den Destillationsböden vorliegenden Maleinsäureanhydrids, wodurch die Endproduktausbeute um ungefähr 3 Prozent verbessert wird.

Dem Fachmann auf diesem Gebiet ist es offensichtlich, dass die obige Beschreibung nur beispielhaft für eine bevorzugte Ausbildungsform des erfindungsgemässen Verfahrens bzw. der Vorrichtung ist, wobei verschiedene Abänderungen möglich sind, die sämtlich in den Schutzbereich der hier beschriebenen und beanspruchten Erfindung fallen.

Ansprüche

1.    Verfahren zur Maleinsäureanhydridgewinnung aus den Abgasen von Phthalsäureanhydridanlagen, bei dem diese Gase hauptsächlich aus Maleinsäureanhydrid, Phthalsäureanhydrid, Citraconsäureanhydrid und Chinonen bestehende organische Substanzen enthalten und welches eine Wäsche dieser Gase zur Abscheidung jener organischen Substanzen in Form einer wässrigen Lösung der entsprechenden Säuren und die Gewinnung von Maleinsäureanhydrid aus dieser sauren Lösung umfasst, dadurch gekennzeichnet, dass man stufenweise:

a) diese saure Lösung durch Zusatz eines an Maleinsäure angereicherten und an Phthalsäure verarmten Rückführungsstroms in jene Waschstufe an Maleinsäure bis zu einem Maleinsäure/Phthalsäuregewichtsverhältnis von 17:1 bis 20:1 und zu einer Maleinsäurekonzentration von 25 - 35 Gew.-% bezogen auf diese saure Lösung anreichert,

b) die Phthalsäure durch Vakuumkristallisation und Filtrieren aus dieser angereicherten Lösung entfernt,

c) einen Teil der aus Schritt (b) kommenden angereicherten Lösung als solchen Rückführungsstrom in den Schritt (a) zurückleitet,

d) den verbleibenden Teil der angereicherten Lösung aus Schritt (b) einer Behandlung zur Gewinnung von Maleinsäureanhydrid unterzieht und

e) das in Schritt (d) erhaltene Maleinsäureanhydrid reinigt.

2.    Verfahren nach Anspruch 1, dadurch gekennzeichnet, dass die Behandlung zur Gewinnung von Maleinsäureanhydrid in Schritt (d) darin besteht, dass man

i) diese angereicherte Lösung durch azeotrope Destillation mit ortho-Xylol bei einer Temperatur von $110^{\circ}$C und Atmosphärendruck dehydratisiert und

ii) das ortho-Xylol durch Vakuumdestillation bei einem Druck von 250 - 300 Torr und einer Kopftemperatur von etwa $110^{\circ}$C entfernt, wobei man rohes Maleinsäureanhydrid als Rückstand erhält.

3.    Verfahren nach Anspruch 2, dadurch gekennzeichnet, dass man das in Schritt (i) abdestillierte Wasser/ortho-Xylolazeotrop kondensiert und sich scheiden lässt, die erhaltene ortho-Xylolphase als Rückfluss in die Schritte (i) und (ii) sowie die wässrige Phase in jene Gaswaschstufe zurückleitet.

4.     Verfahren nach Anspruch 2 oder 3, dadurch gekennzeichnet, dass man das in Schritt (ii) abdestillierte ortho-Xylol kondensiert und mindestens teilweise als Rückfluss in den Schritt (i) zurückleitet.

5.     Verfahren nach einem der vorhergehenden Ansprüche, dadurch gekennzeichnet, dass jene Reinigungsstufe darin besteht, dass man

iii) dieses Rohmaleinsäureanhydrid einer Rektifizierung in Bodenkolonnen in zwei hintereinandergeschalteten Stufen unterzieht, wobei das Maleinsäureanhydrid am Kopf dieser zweiten Stufe bei 100 Torr Druck und einer Temperatur von $135^{\circ}C$ abgezogen und kondensiert wird, was Maleinsäureanhydrid einer Reinheit von über 99,5% (typischerweise 99,8) ergibt.

6.     Verfahren nach Anspruch 5, dadurch gekennzeichnet, dass bei der zweistufigen Rektifizierung in Schritt (iii) das Kopfprodukt der zweiten Stufe kondensiert und teilweise als Rückfluss in diese zurückgeführt und die Sumpfflüssigkeit aus dieser zweiten Stufe als Rückfluss in jene erste Stufe zurückgeführt wird.

7.     Verfahren nach Anspruch 5 oder 6, dadurch gekennzeichnet, dass man bei dieser Destillation des Rohmaleinsäureanhydrids vor diesem selbst eine Fraktion leichtsiedender Produkte abzieht, die in jene Behandlung zur Gewinnung von Maleinsäureanhydrid in Schritt (d) zurückgeführt wird.

8.     Verfahren nach Anspruch 5, 6 oder 7, dadurch gekennzeichnet, dass man diese Rektifizierung mit von 3 - 3,5 auf 9 - 11 ansteigenden Rücklaufverhältnissen durchführt.

9.     Verfahren nach einem der Ansprüche 5 bis 8, dadurch gekennzeichnet, dass man diese Rektifizierung mit mindestens 15 Böden in der ersten Stufe und mindestens 30 Böden in der zweiten Stufe durchführt.

10.     Verfahren nach einem der Ansprüche 5 bis 9, da-

- 14 -                    0119301

durch gekennzeichnet, dass man am Ende dieser Rektifizierung die auf den Böden der ersten Stufe verbliebene Flüssigkeit in den Sumpf der zweiten Stufe ablässt, um das restliche Maleinsäureanhydrid in einem späteren Aufbereitungszyklus zurückzugewinnen.

11. Verfahren nach einem der Ansprüche 5 bis 10, dadurch gekennzeichnet, dass man die bei der Rektifizierung in der ersten Stufe aufgelaufenen Rückstände durch Waschen mit wässriger Lauge entfernt.

12. Verfahren nach einem der Ansprüche 1 bis 11, dadurch gekennzeichnet, dass man die Kristallisation der Phthalsäure in Schritt (b) bei 10 - 15 Torr und etwa $10^{\circ}C$ durchführt.

13. Verfahren zur Maleinsäureanhydridgewinnung aus den Abgasen von Phthalsäureanhydridanlagen, bei dem diese Gase hauptsächlich aus Maleinsäureanhydrid, Phthalsäureanhydrid, Chinonen und Citraconsäureanhydrid bestehende organische Substanzen enthalten und welches eine Wäsche dieser Gase zur Abscheidung jener organischen Substanzen in Form einer wässrigen Lösung der entsprechenden Säuren und die Gewinnung von Maleinsäureanhydrid aus dieser sauren Lösung umfasst, dadurch gekennzeichnet, dass man stufenweise:

a) diese saure Lösung durch Zusatz eines an Maleinsäure angereicherten und an Phthalsäure verarmten Rückführungsstroms in jene Waschstufe an Maleinsäure bis zu einem Maleinsäure/Phthalsäuregewichtsverhältnis von 17:1 bis 20:1 und zu einer Maleinsäurekonzentration von 25 - 35 Gew.-% bezogen auf diese saure Lösung anreichert,

b) die Phthalsäure aus dieser angereicherten Lösung durch Vakuumkristallisation bei einem Druck von 10 - 15 Torr und einer Temperatur von $10^{\circ}C$ und nachfolgendes Filtrieren der erhaltenen Lösung entfernt,

c) einen Teil der aus Schritt (b) kommenden angereicherten Lösung als solchen Rückführungsstrom in den Schritt (a) zurückleitet,

d) die verbleibende angereicherte Lösung aus Schritt (b) einer Behandlung zur Gewinnung von Maleinsäureanhydrid unterzieht, welche darin besteht, dass man

i) diese angereicherte Lösung durch azeotrope Destillation mit ortho-Xylol bei einer Temperatur von 110°C und Atmosphärendruck dehydratisiert und

ii) das ortho-Xylol durch Vakuumdestillation bei einem Druck von 250 - 300 Torr und einer Kopftemperatur von 110°C entfernt, wobei man rohes Maleinsäureanhydrid als Rückstand erhält, und

e) das rohe Maleinsäureanhydrid durch eine Rektifizierungsstufe (iii) in Bodenkolonnen in zwei hintereinandergeschalteten Stufen reinigt, wobei das Maleinsäureanhydrid am Kopf dieser zweiten Stufe bei 100 Torr Druck und einer Temperatur von 135°C abgezogen und kondensiert wird, was Maleinsäureanhydrid einer Reinheit von über 99,5% ergibt.

14. Vorrichtung zur Maleinsäureanhydridgewinnung aus den Abgasen von Phthalsäureanhydridanlagen, bestehend aus einem Gaswaschteil, einem Maleinsäureanhydridgewinnungsteil und einem Maleinsäureanhydridreinigungsteil, dadurch gekennzeichnet, dass

A) dieser Waschteil mindestens eine Waschkolonne zur Gegenstromwäsche dieser Gase mit wässriger Lösung und rückgeführter, an Maleinsäure angereicherter Lösung, mindestens einen mit Evakuierungsmitteln und Rührwerk versehenen Kristallisator zur Kristallisation der Phthalsäure und mindestens ein Vakuumfilter zur Entfernung der kristallisierten Phthalsäure sowie Einrichtungen zur teilweisen Rückführung der aus dem Filter austretenden, angereicherten Lösung in jene Waschkolonne umfasst,

B) dieser Maleinsäureanhydridgewinnungsteil mindestens eine Destillationskolonne mit Böden, einen Kessel vor dieser Kolonne und einen Kondensator sowie eine Vorlage nach dieser Kolonne und Mittel zum Evakuieren dieser Kolonne umfasst und

C) dieser Maleinsäureanhydridreinigungsteil mindestens zwei hintereinandergeschaltete Rektifizierkolonnen mit Böden, mindestens eine Pumpe zur Förderung von Sumpfprodukt aus der zweiten Kolonne von diesen als Rückfluss auf den Kopf dieser ersten Rektifizierkolonne, Mittel zum Evakuieren dieser Rektifizierkolonnen und einen Kessel

0119301

vor sowie einen Kondensator und eine Vorlage nach diesen
Rektifizierkolonnen umfasst.

15. Vorrichtung nach Anspruch 14, dadurch gekennzeichnet, dass diese Waschkolonne Böden und Füllkörper enthält,
die innigsten Kontakt der Gase mit den wässrigen Waschlösungen bei geringster Verschmutzung mit ausgefällten
Substanzen und bei kleinstem Druckverlust ermöglichen.

16. Vorrichtung nach Anspruch 14 oder 15, dadurch
gekennzeichnet, dass diese Waschzone ferner vor jenem Filter einen Homogenisator zum Speichern dieser sauren Lösung,
falls die Filtration diskontinuierlich betrieben wird,
enthält.

17. Vorrichtung nach einem der Ansprüche 14 bis 16,
dadurch gekennzeichnet, dass jene Destillationskolonne
des besagten Gewinnungsteils (B) auch die Funktion dieser
ersten Rektifizierkolonne des Reinigungsteils (C) übernimmt und dass dieser Kessel, dieser Kondensator und diese
Vorlage sowie jene Mittel zum Evakuieren diesen beiden
Teilen, der Gewinnung (B) und der Reinigung (C), gemeinsam sind.

18. Vorrichtung nach einem der Ansprüche 14 bis 17,
dadurch gekennzeichnet, dass diese Destillationskolonne
und/oder diese erste Rektifizierkolonne mindestens 15
Böden und diese zweite Rektifizierkolonne mindestens 30
Böden aufweisen.

19. Vorrichtung nach einem der Ansprüche 14 bis 18,
dadurch gekennzeichnet, dass diese Destillationskolonne
und/oder diese erste Rektifizierkolonne aus titanhaltigen
Werkstoffen gefertigt sind.

20. Verfahren zur Maleinsäureanhydridgewinnung aus
den Abgasen von Phthalsäureanhydridanlagen, wie beschrieben und veranschaulicht.

21. Vorrichtung zur Maleinsäureanhydridgewinnung aus
den Abgasen von Phthalsäureanhydridanlagen, wie beschrieben und veranschaulicht.

PHTHAL-SAEURE

MALEINSAEURE-LOESUNG

AUSTRITT DER GEWASCHENEN GASE

Fig.1

Fig.2

ZUM
SKRUBBER

KUEHLWASSER

WARMES WASSER

KUEHLWASSER

WARMES WASSER

24

19

24

14

11

ZUM
SKRUBBER

22

20

21

WASCHWASSER
UND LAUGE

ORTHO-XYLOL

MALEINSAEUREANHYDRID-
HALTIGE LEICHTE FRAKTION

DAMPF

FC

17

18

26

o-XYLOL

MALEINSAEURE-
ANHYDRIDHALTIGE
LEICHTE FRAKTION

REINES
MALEIN-
SAEURE-
ANHYDRID

23

LC

25

16

15

28

27

25

KONDENSAT

0119301